# EUROPEAN PATENT APPLICATION

(11) **EP 1 435 393 A1**
(43) Date of publication of application: **07.07.2004**
(21) Application number: 03028473.1
(22) Date of filing: 12.12.2003
(51) Int. Cl.: C12Q 1/68

(54) **Method for analyzing gene expression levels by quantification of pyrophosphoric acid**

(30) Priority: 12.12.2002 JP 2002360915
(71) Applicant: FUJI PHOTO FILM CO., LTD., Kanagawa-ken, 250-0193 (JP)
(72) Inventor: Iwaki, Yoshihide, c/o Fuji Photo Film Co.,Ltd., Asaka-shi, Saitama 351-8585 (JP); Makino, Yoshihiko, Asaka-shi, Saitama 351-8585 (JP); Inomata, Hiroko, Asaka-shi, Saitama 351-8585 (JP)
(74) Representative: Albrecht, Thomas, Dr.

(57) **Abstract**

An object of the present invention to provide a method for analyzing gene expression levels which can be simply and rapidly carried out by using a small apparatus without requiring any special techniques, complicated operations and special apparatuses. The present invention provides a method for analyzing expression levels of a target nucleic acid in a biological sample which comprises steps of: (1) conducting polymerase elongation by reacting RNA from a biological sample or cDNA synthesized therefrom, at least one primer complementary with a part of the target nucleic acid sequence contained in the RNA, at least one deoxynucleoside triphosphate, and at least one polymerase to synthesize said target nucleic acid sequence; and (2) detecting or quantifying pyrophosphoric acid which is generated upon the polymerase elongation.

SEQUENCE LISTING

<110> FUJI PHOTO FILM CO., LTD.

<120> A METHOD FOR ANALYZING EXPRESSION LEVELS OF GENE

<130> 13826EP

<140>
<141>

<150> JP 360915/2002
<151> 2002-12-12

<160> 4

<170> PatentIn Ver. 2.1

<210> 1
<211> 21
<212> DNA
<213> Artificial Sequence

<220>
<223> Description of Artificial Sequence:Primer

<400> 1
gaaaagtggg cagcaaatgt t                                                21

<210> 2
<211> 20
<212> DNA
<213> Artificial Sequence

<220>
<223> Description of Artificial Sequence:Primer

<400> 2
tcctcggcaa agcaggtctc                                                  20

<210> 3
<211> 21
<212> DNA
<213> Artificial Sequence

<220>

<223> Description of Artificial Sequence:Primer

<400> 3
tgggacgaca tggagaaaat c                                                21

<210> 4
<211> 21
<212> DNA
<213> Artificial Sequence

<220>
<223> Description of Artificial Sequence:Primer

<400> 4
aggaaggaag gctggaagag t                                                21

## Description

### Technical Field

The present invention relates to a method for analyzing expression levels of gene, and more particularly to a method for analyzing expression levels of the target nucleic acid in a biological sample.

### Background Art

Northern blotting, RT-PCR, the microarray technique and other techniques are known as techniques for assaying differences in gene expression levels between two types of cells (e.g., between a normal cell and a cancerous cell). Northern blotting and RT-PCR are employed in order to verify the data that was cyclopaedically analyzed by the microarray technique. In Northern blotting, RNA prepared from a sample is blotted on a membrane or the like, and detection is carried out using a labeled probe. In this technique, preparation of a labeled probe or detection by hybridization is relatively complicated. In conventional RT-PCR, cDNA was synthesized using reverse transcriptase from RNA prepared from a sample, PCR was carried out using this cDNA as a template and using a primer specific to the target gene, and the amplified product was subjected to electrophoresis. Thus, the level of the target gene expression in a sample was assayed. With this technique, however, the assay of the amount of the amplified product by electrophoresis was often conducted visually, and quantitative assay was difficult.

### Summary of the Invention

An object of the present invention is to provide a means for quantitatively assaying the levels of the target gene expression that alternates a conventional technique of observing the amplified product by electrophoresis when assaying the gene expression levels by RT-PCR. Further, it is another object of the present invention to provide a method for analyzing gene expression levels which can be simply and rapidly carried out by using a small apparatus without requiring any special techniques, complicated operations and special apparatuses.

The present inventors have conducted concentrated studies in order to solve the above objects. As a result, they have found that gene expression levels can be simply and rapidly analyzed, without requiring any special apparatus, by detecting and assaying pyrophosphoric acid which is generated upon polymerase elongation that utilizes RNA from a biological sample or cDNA synthesized therefrom as a template by, preferably, using a dry analytical element. This has led to the completion of the present invention.

Thus, the present invention provides a method for analyzing expression levels of a target nucleic acid in a biological sample which comprises steps of:
(1) conducting polymerase elongation by reacting RNA from a biological sample or cDNA synthesized therefrom, at least one primer complementary with a part of the target nucleic acid sequence contained in the RNA, at least one deoxynucleoside triphosphate, and at least one polymerase to synthesize said target nucleic acid sequence; and
(2) detecting or quantifying pyrophosphoric acid which is generated upon the polymerase elongation.

Preferably, the expression level of the target nucleic acid is analyzed by using RNA from two or more samples or cDNA synthesized therefrom.

Preferably, the expression level of the target nucleic acid is analyzed by using a gene that ubiquitously exists in every cell as an internal standard and normalizing the expression level of the target nucleic acid by using it.

Preferably, β-actin or GAPDH gene is used as the internal standard.

Preferably, pyrophosphoric acid is detected by using colorimetry.

Preferably, pyrophosphoric acid is detected by using a dry analytical element.

Preferably, the dry analytical element is the one for pyrophosphoric acid quantification, which comprises a reagent layer containing xanthosine or inosine, pyrophosphatase, purine nucleoside phosphorylase, xanthine oxidase, peroxidase, and a color developer.

Preferably, the polymerase is selected from the group consisting of DNA polymerase I, Klenow fragment of DNA polymerase I, Bst DNA polymerase, and reverse transcriptase.

Preferably, pyrophosphoric acid is detected by enzymatically converting pyrophosphoric acid to inorganic phosphorus and then using a dry analytical element for inorganic phosphorus quantification which comprises a reagent layer containing xanthosine or inosine, purine nucleoside phosphorylase, xanthine oxidase, peroxidase, and a color developer. In this case, the enzyme that converts pyrophosphoric acid to inorganic phosphorus is pyrophosphatase. In this case, the polymerase is preferably selected from the group consisting of DNA polymerase I, Klenow fragment of DNA polymerase I, Bst DNA polymerase, and reverse transcriptase.

### Brief Description of the Drawing

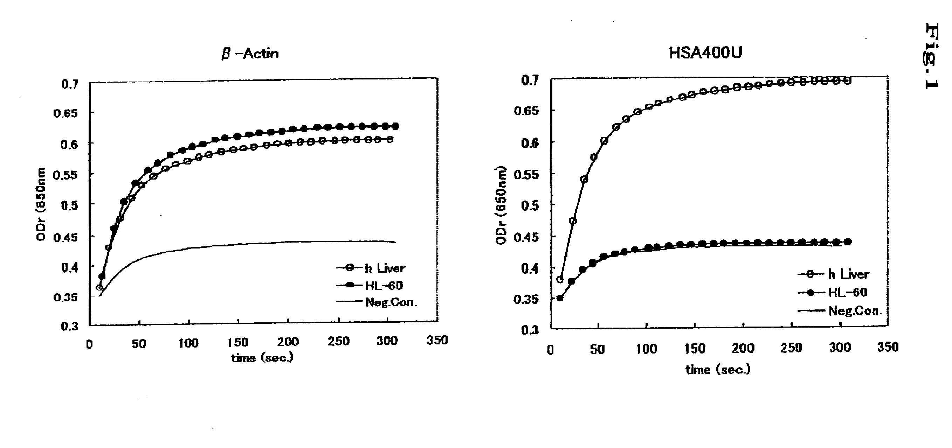

Fig. 1 shows changes in OD_{R} with time when the PCR solution of the target gene of each cell is spotted on a slide.

### Best Mode for Carrying out the Invention

In a preferable embodiment of the method for analyzing the expression levels of the target nucleic acid according to the present invention, pyrophosphoric acid is analyzed by using colorimetry. More preferably, pyrophosphoric acid is detected by using a dry analytical element. According to the method for analysis of the present invention, the occurrence of the expression of the target nucleic acid can be detected and the expression level of the target nucleic acid can be assayed.

The first preferred embodiment of the method for analyzing expression levels of a target nucleic acid in a biological sample according to the present invention is listed below.
(i) Detection of pyrophosphoric acid is carried out by using a dry analytical element for quantifying pyrophosphoric acid which comprises a reagent layer containing xanthosine or inosine, pyrophosphatase, purine nucleoside phosphorylase, xanthine oxidase, peroxidase, and a color developer.
(ii) Polymerase to be used is selected from the group consisting of DNA polymerase I, Klenow fragment of DNA polymerase I, Bst DNA polymerase, and reverse transcriptase.

The second preferred embodiment of the present invention is characterized in that the detection of pyrophosphoric acid which is generated upon the polymerase elongation reaction is carried out by enzymatically converting pyrophosphoric acid into inorganic phosphorous, followed by the use of a dry analytical element for quantifying inorganic phosphorus which comprises a reagent layer containing xanthosine or inosine, purine nucleoside phosphorylase, xanthine oxidase, peroxidase, and a color developer.

Preferred embodiments of a method for analyzing expression levels of a target nucleic acid in a biological sample according to the second aspect of the present invention are listed below.
(i) Pyrophosphatase is used as an enzyme for converting pyrophosphoric acid.
(ii) Polymerase to be used is selected from the group consisting of DNA polymerase I, Klenow fragment of DNA polymerase I, Bst DNA polymerase, and reverse transcriptase.

The embodiments of the present invention will be described in more detail in the following.

### (A) Target nucleic acid fragment:

A target nucleic acid fragment in the present invention is one which is contained in RNA from at least 2 types of samples, and is a sequence of a gene of which expression level is assayed by the present invention.

The types of the sample used in the present invention is not particularly limited, and any sample from any living body such as animal, microorganisms, bacteria, and plants.

A target nucleic acid fragment is a polynucleotide, where at least a part of its nucleotide sequence is known.

In the present invention, polymerase elongation (preferably PCR) is preferably conducted by using RNA from at least two types of samples or cDNA synthesized therefrom as a template. When RNA is used, cDNA may be synthesized by performing polymerase elongation using reverse transcriptase. Further, this cDNA may be used as a template to conduct polymerase elongation (preferably PCR).

Preferably, RNA from samples or cDNA synthesized therefrom is purified as much as possible, and unnecessary components other than the nucleic acid (particularly a component that inhibits polymerase elongation) are removed therefrom.

### (B) Primer complementary with target nucleic acid fragment:

A primer complementary with a target nucleic acid fragment used in the present invention is oligonucleotide having a nucleotide sequence complementary with a target site, the nucleotide sequence of the target nucleic acid fragment being known. Hybridization of a primer complementary with the target nucleic acid fragment to a target site of the target nucleic acid fragment results in progress on polymerase elongation reaction starting from the 3' terminus of the primer and using the target nucleic acid as template. Thus, whether or not the primer recognizes and specifically hybridizes to a target site of the target nucleic acid fragment is an important issue in the present invention. The number of nucleotides in the primer used in the present invention is preferably 5 to 60, and particularly preferably 15 to 40. If the number of nucleotides in the primer is too small, specificity with the target site of the target nucleic acid fragment is deteriorated and also a hybrid with the target nucleic acid fragment cannot be stably formed. When the number of nucleotides in the primer is too high, double-strands are disadvantageously formed due to hydrogen bonds between primers or between nucleotides in a primer. This also results in deterioration in specificity.

When the existence of the target nucleic acid fragment is detected by the method according to the present invention, a plurality of primers complementary with each different site in the target nucleic acid fragment can be used. Thus, recognition of the target nucleic acid fragment in a plurality of sites results in improvement in specificity in detecting the existence of the target nucleic acid fragment. When a part of the target nucleic acid fragment is amplified (e.g., PCR), a plurality of primers can be designed in accordance with the amplification methods.

When the expression of the target nucleic acid is detected by the method according to the present invention, a primer is designed so as to contain a portion of the target nucleic acid of interest. Thus, the occurrence of expression of the target nucleic acid causes difference in the occurrence of hybridization of the primer to the target nucleic acid fragment, and this eventually enables the assay of the expression level as difference in polymerase elongation reaction.

### (C) Polymerase:

When the target nucleic acid is DNA, polymerase used in the present invention is DNA polymerase which catalyzes complementary elongation reaction which starts from the double-strand portion formed by hybridization of the primer with the target nucleic acid fragment in its portion denatured into single-strand in the 5' → 3' direction by using deoxynucleoside triphosphate (dNTP) as material and using the target nucleic acid fragment as template. Specific examples of DNA polymerase used include DNA polymerase I, Klenow fragment of DNA polymerase I, and Bst DNA polymerase. DNA polymerase can be selected or combined depending on the purpose. For example, when a part of the target nucleic acid fragment is amplified (e.g., PCR), use of Taq DNA polymerase which is excellent in heat resistance, is effective. When a part of the target nucleic acid fragment is amplified by using the amplification method (loop-mediated isothermal amplification of DNA (the LAMP method)) described in "BIO INDUSTRY, Vol. 18, No. 2, 2001," use of Bst DNA polymerase is effective as strand displacement-type DNA polymerase which has no nuclease activity in the 5' → 3' direction and catalyzes elongation reaction while allowing double-strand DNA to be released as single-strand DNA on the template. Use of DNA polymerase α, T4 DNA polymerase, and T7 DNA polymerase, which have hexokinase activity in the 3' → 5' direction in combination is also possible depending on the purpose.

When mRNA is a target nucleic acid fragment, reverse transcriptase having reverse transcription activity can be used. Further, reverse transcriptase can be used in combination with Taq DNA polymerase, or an enzyme having reverse transcriptase activity and DNA polymerase activity in combination may be used. These enzymes are used in the case of RT-PCR which is a preferred embodiment of the present invention.

### (D) Polymerase elongation reaction:

Polymerase elongation reaction in the present invention includes all the elongation processes of complementary nucleic acids. These elongation processes proceed by starting from the 3' terminus of a primer complementary to the target nucleic acid fragment as described in (B) above, which was specifically hybridized to a part of the region of the target nucleic acid fragment which was denatured into a single strand as described in (A). Also, deoxynucleoside triphosphates (dNTP) are used as components, the polymerase as described in (C) above is used as a catalyst, and the target nucleic acid fragment is used as a template. This elongation reaction of complementary nucleic acids indicates that continuous elongation reaction occurs at least twice (corresponding to 2 nucleotides).

Examples of a representative polymerase elongation reaction and an amplification reaction of a subject site of the target nucleic acid fragment involving polymerase elongation reaction are shown below. The simplest case is that only one polymerase elongation reaction in the 5' → 3' direction is carried out using the target nucleic acid fragment as template. This polymerase elongation reaction can be carried out under isothermal conditions. In this case, the amount of pyrophosphoric acid generated as a result of polymerase elongation reaction is in proportion to the initial amount of the target nucleic acid fragment. Specifically, it is a suitable method for quantitatively detecting the existence of the target nucleic acid fragment.

When the amount of the target nucleic acid is small, a target site of the target nucleic acid is preferably amplified by any means utilizing polymerase elongation reaction. In the amplification of the target nucleic acid, various methods which have been heretofore developed, can be used. The most general and spread method for amplifying the target nucleic acid is polymerase chain reaction (PCR). PCR is a method of amplifying a target portion of the target nucleic acid fragment by repeating periodical processes of denaturing (a step of denaturing a nucleic acid fragment from double-strand to single-strand) → annealing (a step of hybridizing a primer to a nucleic acid fragment denatured into single-strand) → polymerase (Taq DNA polymerase) elongation reaction → denaturing, by periodically controlling the increase and decrease in temperature of the reaction solution. Finally, the target site of the target nucleic acid fragment can be amplified 1,000,000 times as compared to the initial amount. Thus, the amount of accumulated pyrophosphoric acid generated upon polymerase elongation reaction in the amplification process in PCR becomes large, and thereby the detection becomes easy.

A cycling assay method using exonuclease described in Japanese Patent Publication Laying-Open No. 5-130870 is a method for amplifying a target site of the target nucleic acid fragment utilizing polymerase elongation. In this method, a primer is decomposed from a reverse direction by performing polymerase elongation reaction starting from a primer specifically hybridized with a target site of the target nucleic acid fragment, and allowing 5' *→* 3' exonuclease to act. In place of the decomposed primer, a new primer is hybridized, and elongation reaction by DNA polymerase proceeds again. This elongation reaction by polymerase and the decomposition reaction by exonuclease for removing the previously elongated strand are successively and periodically repeated. The elongation reaction by polymerase and the decomposition reaction by exonuclease can be carried out under isothermal conditions. The amount of accumulated pyrophosphoric acid generated in polymerase elongation reaction repeated in this cycling assay method becomes large, and the detection becomes easy.

The LAMP method is a recently developed method for amplifying a target site of the target nucleic acid fragment. This method is carried out by using at least 4 types of primers, which complimentarily recognize at least 6 specific sites of the target nucleic acid fragment, and strand displacement-type Bst DNA polymerase, which has no nuclease activity in the 5' → 3' direction and which catalyzes elongation reaction while allowing the double-strand DNA on the template to be released as single-strand DNA. In this method, a target site of the target nucleic acid fragment is amplified as a special structure under isothermal conditions. The amplification efficiency of the LAMP method is high, and the amount of accumulated pyrophosphoric acid generated upon polymerase elongation reaction is very large, and the detection becomes easy.

In a preferred embodiment of the present invention, the target nucleic acid fragment is a RNA fragment, and elongation reaction is carried out by using reverse transcriptase having reverse transcription activity and using the RNA strand as template. Further, RT-PCR can be utilized where reverse transcriptase is used in combination with Taq DNA polymerase, and reverse transcription (RT) reaction is carried out, followed by PCR. An enzyme having reverse transcription activity and DNA polymerase activity in combination can also be used here. Detection of pyrophosphoric acid generated in the RT reaction or RT-PCR reaction enables the detection of the existence of the RNA fragment of the target nucleic acid fragment.

### (E) Detection of pyrophosphoric acid (PPi):

A method represented by formula 1 has been heretofore known as a method for detecting pyrophosphoric acid (PPi). In this method, pyrophosphoric acid (PPi) is converted into adenosinetriphosphate (ATP) with the aid of sulfurylase, and luminescence generated when adenosinetriphosphate acts on luciferin with the aid of luciferase is detected. Thus, an apparatus capable of measuring luminescence is required for detecting pyrophosphoric acid (PPi) by this method.

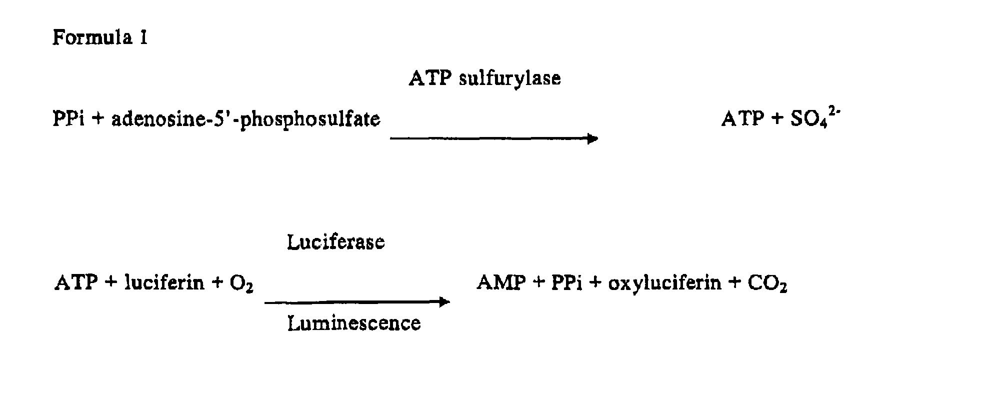

A method for detecting pyrophosphoric acid suitable for the present invention is a method represented by formula 2 or 3. In the method represented by formula 2 or 3, pyrophosphoric acid (PPi) is converted into inorganic phosphate (Pi) with the aid of pyrophosphatase, inorganic phosphate (Pi) is reacted with xanthosine or inosine with the aid of purine nucleoside phosphorylase (PNP), the resulting xanthine or hypoxanthine is oxidated with the aid of xanthine oxidase (XOD) to generate uric acid, and a color developer (a dye precursor) is allowed to develop color with the aid of peroxidase (POD) using hydrogen peroxide (H₂O₂) generated in the oxidation process, followed by colorimetry. In the method represented by formula 2 or 3, the result can be detected by colorimetry and, thus, pyrophosphoric acid (PPi) can be detected visually or using a simple colorimetric measuring apparatus.

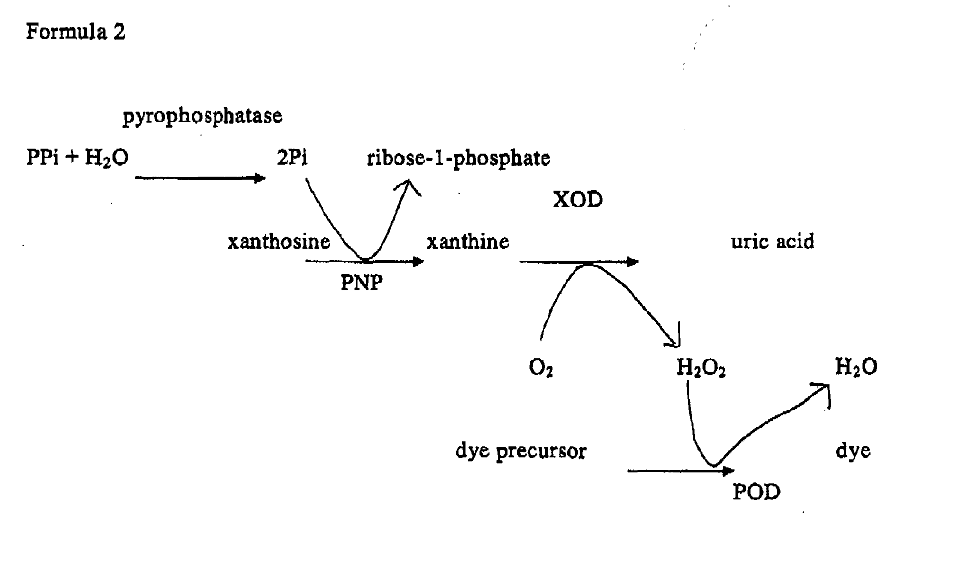

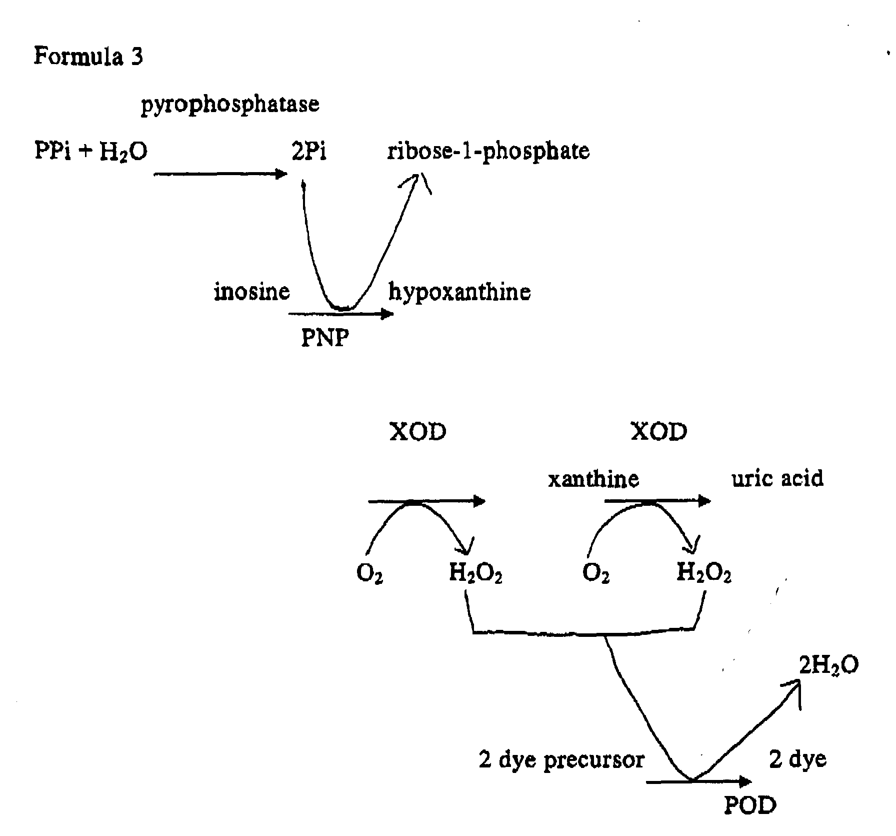

Commercially available pyrophosphatase (EC3, 6, 1, 1), purine nucleoside phosphorylase (PNP, EC2. 4. 2. 1), xanthine oxidase (XOD, EC1. 2. 3. 2), and peroxidase (POD, EC1. 11. 1. 7) can be used. A color developer (i.e., a dye precursor) may be any one as long as it can generate a dye by hydrogen peroxide and peroxidase (POD), and examples thereof which can be used herein include: a composition which generates a dye upon oxidation of leuco dye (e.g., triarylimidazole leuco dye described in U.S.Patent. No. 4,089,747 and the like, diarylimidazole leuco dye described in Japanese Patent Publication Laying-Open No. 59-193352 (EP 0122641A)); and a composition (e.g., 4-aminoantipyrines and phenols or naphthols) containing a compound generating a dye by coupling with other compound upon oxidation.

### (F) Dry analytical element:

A dry analytical element which can be used in the present invention is an analytical element which comprises a single or a plurality of functional layers, wherein at least one layer (or a plurality of layers) comprises a detection reagent, and a dye generated upon reaction in the layer is subjected to quantification by colorimetry by reflected light or transmitted light from the outside of the analytical element.

In order to perform quantitative analysis using such a dry analytical element, a given amount of liquid sample is spotted onto the surface of a developing layer. The liquid sample spread on the developing layer reaches the reagent layer and reacts with the reagent thereon and develops color. After spotting, the dry analytical element is maintained for a suitable period of time at given temperature (for incubation) and a color developing reaction is allowed to thoroughly proceed. Thereafter, the reagent layer is irradiated with an illuminating light from, for example, a transparent support side, the amount of reflected light in a specific wavelength region is measured to determine the optical density of reflection, and quantitative analysis is carried out based on the previously determined calibration curve.

Since a dry analytical element is stored and kept in a dry state before detection, it is not necessary that a reagent is prepared for each use. As stability of the reagent is generally higher in a dry state, it is better than a so-called wet process in terms of simplicity and swiftness since the wet process requires the preparation of the reagent solution for each use. It is also excellent as an examination method because highly accurate examination can be swiftly carried out with a very small amount of liquid sample.

### (G) Dry analytical element for quantifying pyrophosphoric acid:

A dry analytical element for quantifying pyrophosphoric acid which can be used in the present invention can have a layer construction which is similar to various known dry analytical elements. The dry analytical element may be multiple layers which contain, in addition to a reagent for performing the reaction represented by formula 2 or 3 according to item (E) above (detection of pyrophosphoric acid (PPi)), a support, a developing layer, a detection layer, a light-shielding layer, an adhesive layer, a water-absorption layer, an undercoating layer, and other layers. Examples of such dry analytical elements include those disclosed in the specifications of Japanese Patent Publication Laying-Open No. 49-53888 (U.S. Patent. No. 3,992,158), Japanese Patent Publication Laying-Open No. 51-40191 (U.S. Patent. No. 4,042,335), Japanese Patent Publication Laying-Open No. 55-164356 (U.S.Patent. No. 4,292,272), and Japanese Patent Publication Laying-Open No. 61-4959 (EPC Publication No. 0166365A).

Examples of the dry analytical element to be used in the present invention include a dry analytical element for quantifying pyrophosphoric acid which comprises a reagent for converting pyrophosphoric acid into inorganic phosphorus and a reagent layer containing a group of reagent for carrying out a coloring reaction depending of the amount of inorganic phosphorus.

In this dry analytical element for quantitative assay of pyrophosphate, pyrophosphoric acid (PPi) can enzymatically be converted into inorganic phosphorus (Pi) using pyrophosphatase as described above. The subsequent process, that is color reaction depending on the amount of inorganic phosphorus (Pi), can be performed using "quantitative assay method of inorganic phosphorus" (and combinations of individual reactions used therefor), described hereinafter, which is known in the field of biochemical inspection.

It is noted that when representing "inorganic phosphorus," both the expressions "Pi" and "HPO₄²⁻, H₂PO₄¹⁻" are used for phosphoric acid (phosphate ion). Although the expression "Pi" is used in the examples of reactions described below, the expression "HPO₄²⁻" may be used for the same reaction formula.

As the quantitative assay method of inorganic phosphorus, an enzyme method and a phosphomolybdate method are known. Hereinafter, this enzyme method and phosphomolybdate method will be described as the quantitative assay method of inorganic phosphorus.

### A. Enzyme method

Depending on the enzyme to be used for the last color reaction during a series of reactions for Pi quantitative detection, the following methods for quantitative assay are available: using peroxidase (POD); or using glucose-6-phosphate dehydrogenase (G6PDH), respectively. Hereinafter, examples of these methods are described.

### (1) Example of the method using peroxidase (POD)

### (1-1)

Inorganic phosphorus (Pi) is allowed to react with inosine by purine nucleoside phosphorylase (PNP), and the resultant hypoxanthine is oxidized by xanthine oxidase (XOD) to produce uric acid. During this oxidization process, hydrogen peroxide (H₂O₂) is produced. Using the thus produced hydrogen peroxide, 4-aminoantipyrines (4-AA) and phenols are subjected to oxidization-condensation by peroxidase (POD) to form a quinonimine dye, which is colorimetrically assessed.

### (1-2)

Pyruvic acid is oxidized by pyruvic oxidase (POP) in the presence of inorganic phosphorus (Pi), cocarboxylase (TPP), flavin adenine dinucleotide (FAD) and Mg²⁺ to produce acetyl acetate. During this oxidization process, hydrogen peroxide (H₂O₂) is produced. Using the thus produced hydrogen peroxide, 4-aminoantipyrines (4-AA) and phenols are subjected to oxidization-condensation by peroxidase (POD) to form a quinonimine dye which is colorimetrically assessed, in the same manner as described in (1-1).

It is noted that the last color reaction for each of the above processes (1-1) and (1-2) can be performed by a "Trinder reagent" which is known as a detection reagent for hydrogen peroxide. In this reaction, phenols function as "hydrogen donors." Phenols to be used as "hydrogen donors" are classical, and now various modified "hydrogen donors" are used. Examples of these hydrogen donors include N-ethyl-N-sulfopropyl-m-anilidine, N-ethyl-N-sulfopropylaniline, N-ethyl-N-sulfapropyl-3,5-dimethoxyaniline, N-sulfopropyl-3,5-dimethoxyaniline, N-ethyl-N-sulfopropyl-3,5-dimethylaniline, N-ethyl-N-sulfopropyl-m-toluidine, N-ethyl-N-(2-hydroxy-3-sulfopropyl)-m-anilidine N-ethyl-N-(2-hydroxy-3-sulfopropyl)aniline, N-ethyl-N-(2-hydroxy-3-sulfopropyl)-3,5-dimethoxyaniline, N-(2-hydroxy-3-sulfopropyl)-3,5-dimetboxyaniline, N-ethyl-N-(2-hydroxy-3-sulfopropyl)-3,5-dimethylaniline, N-ethyl-N-(2-hydroxy-3-sulfopropyl)-m-toluidine, and N-sulfogropylaniline.

### (2) Example of a method using glucose-6-phosphate dehydrogenase (G6PDH)

### (2-1)

Inorganic phosphorus (Pi) is reacted with glycogen with phosphorylase to produce glucose-1-phosphate (G-1-P). The produced glucose-1-phosphate is converted into glucose-6-phosphate (G-6-P) with phosphoglucomutase (PGM). In the presence of glucose-6-phosphate and nicotiamide adenine dinucleotide (NAD), NAD is reduced to NADH with glucose-6-phosphate dehydrogenase (G6PDH), followed by colorimetric analysis of the produced NADH.

### (2-2)

Inorganic phosphorus (Pi) is reacted with maltose with maltose phosphorylase (MP) to produce glucose-1-phosphate (G-1-P). Thereafter, the produced glucose-1-phosphate is converted into glucose-6-phosphate (G-6-P) with phosphoglucomutase (PGM) in the same manner as described in (2-1). In the presence of glucose-6-phosphate and nicotiamide adenine dinucleotide (NAD), NAD is reduced to NADH with glucose-6-phosphate dehydrogenase (G6PDH), followed by colorimetric analysis of the produced NADH.

### B. Phosphomolybdate method

There are two phosphomolybdate methods. One is a direct method wherein "Phosphomolybdates (H₃[PO₄Mo₁₂O₃₆])" prepared by complexing inorganic phosphorus (phosphate) and aqueous molybdate ions under acidic condition are directly quantified. The other is a reduction method wherein further to the above direct method, Mo(IV) is reduced to Mo(III) by a reducing agent and molybudenum blue (Mo(III)) is quantified. Examples of the aqueous molybdate ions include aluminum molybdate, cadmium molybdate, calcium molybdate, barium molybdate, lithium molybdate, potassium molybdate, sodium molybdate, and ammonium molybdate. Representative examples of the reducing agents to be used in the reduction method include 1-amino-2-naphthal-4-sulfonic acid, ammonium ferrous sulfate, ferrous chloride, stannous chloride-hydrazine, *p*-methylaminophenol sulfate, N,N-dimethyl-phenylenediamine, ascorbic acid, and malachite green.

When a light-transmissive and water-impervious support is used, the dry analytical element can be practically constructed as below. However, the scope of the present invention is not limited to these.
(1) One having a reagent layer on the support.
(2) One having a detection layer and a reagent layer in that order on the support,
(3) One having a detection layer, a light reflection layer, and a reagent layer in that order on the support.
(4) One having a second reagent layer, a light reflection layer, and a first reagent layer in that order on the support.
(5) One having a detection layer, a second reagent layer, a light reflection layer, and a first reagent layer in that order on the support.

In (1) to (3) above, the reagent layer may be constituted by a plurality of different layers. For example, a first reagent layer may contain enzyme pyrophosphatase which is required in the pyrophosphatase reaction represented by formula 2 or 3, and substrate xanthosine or substrate inosine and enzyme PNP which are required in the PNP reaction, a second reagent layer may contain enzyme XOD which is required in the XOD reaction represented by formula 2 or 3, and a third reagent layer may contain enzyme POD which is required in the POD reaction represented by formula 2 or 3, and a coloring dye (dye precursor). Alternatively, two reagent layers are provided. On the first reagent layer, the pyrophosphatase reaction and the PNP reaction may be proceeded, and the XOD reaction and the POD reaction may be proceeded on the second reagent layer. Alternatively, the pyrophosphatase reaction, the PNP reaction and the XOD reaction may be proceeded on the first reagent layer, and the POD reaction may be proceeded on the second reagent layer.

A water absorption layer may be provided between a support and a reagent layer or detection layer. A filter layer may be provided between each layer. A developing layer may be provided on the reagent layer and an adhesive layer may be provided therebetween.

Any of light-nontransmissive (opaque), light-semitransmissive (translucent), or light-transmissive (transparent) support can be used. In general, a light-transmissive and water-impervious support is preferred. Preferable materials for a light-transmissive and water-impervious support are polyethylene terephthalate or polystyrene. In order to firmly adhere a hydrophilic layer, an undercoating layer is generally provided or hydrophilization is carried out.

When a porous layer is used as a reagent layer, the porous medium may be a fibrous or nonfibrous substance. Fibrous substances used herein include, for example, filter paper, non-woven fabric, textile fabric (e.g. plain-woven fabric), knitted fabric (e.g., tricot knitted fabric), and glass fiber filter paper. Nonfibrous substances may be any of a membrane filter comprising cellulose acetate etc., described in Japanese Patent Publication Laying-Open No. 49-53888 and the like, or a particulate structure having mutually interconnected spaces comprising fine particles of inorganic substances or organic substances described in, for example, Japanese Patent Publication Laying-Open No. 49-53888, Japanese Patent Publication Laying-Open No. 55-90859 (U.S. Patent. No. 4,258,001), and Japanese Patent Publication Laying-Open No. 58-70163 (U.S. Patent. No. 4,486,537). A partially-adhered laminate which comprises a plurality of porous layers described in, for example, Japanese Patent Publication Laying-Open No. 61-4959 (EP Publication 0166365A), Japanese Patent Publication Laying-Open No. 62-116258, Japanese Patent Publication Laying-Open No. 62-138756 (EP Publication 0226465A), Japanese Patent Publication Laying-Open No. 62-138757 (EP Publication 0226465A), and Japanese Patent Publication Laying-Open No. 62-138758 (EP Publication 0226465A), is also preferred.

A porous layer may be a developing layer having so-called measuring action, which spreads liquid in an area substantially in proportion to the amount of the liquid to be supplied. Preferably, a developing layer is textile fabric, knitted fabric, and the like. Textile fabrics and the like may be subjected to glow discharge treatment as described in Japanese Patent Publication Laying-Open No. 57-66359. A developing layer may comprise hydrophilic polymers or surfactants as described in Japanese Patent Publication Laying-Open No. 60-222770 (EP 0162301A), Japanese Patent Publication Laying-Open No. 63-219397 (German Publication DE 3717913A), Japanese Patent Publication Laying-Open No. 63-112999 (DE 3717913A), and Japanese Patent Publication Laying-Open No. 62-182652 (DE 3717913A) in order to regulate a developing area, a developing speed and the like.

For example, a method is useful where the reagent of the present invention is previously impregnated into or coated on a porous membrane etc., comprising paper, fabric or polymer, followed by adhesion onto another water-pervious layer provided on a support (e.g., a detection layer) by the method as described in Japanese Patent Publication Laying-Open No. 55-1645356.

The thickness of the reagent layer thus prepared is not particularly limited. When it is provided as a coating layer, the thickness is suitably in the range of about 1 µm to 50 µm, preferably in the range of 2 µm to 30 µm. When the reagent layer is provided by a method other than coating, such as lamination, the thickness can be significantly varied in the range of several tens of to several hundred µm.

When a reagent layer is constituted by a water-pervious layer of hydrophilic polymer binders, examples of hydrophilic polymers which can be used include: gelatin and a derivative thereof (e.g., phthalated gelatin); a cellulose derivative (e.g., hydroxyethyl cellulose); agarose, sodium arginate; an acrylamide copolymer or a methacrylamide copolymer (e.g., a copolymer of acrylamide or methacrylamide and various vinyl monomers); polyhydroxyethyl methacrylate; polyvinyl alcohol; polyvinyl pyrrolidone; sodium polyacrylate; and a copolymer of acrylic acid and various vinyl monomers.

A reagent layer composed of hydrophilic polymer binders can be provided by coating an aqueous solution or water dispersion containing the reagent composition of the present invention and hydrophilic polymers on the support or another layer such as a detection layer followed by drying the coating in accordance with the methods described in the specifications of Japanese Patent Examined Publication No. 53-21677 (U.S. Patent No. 3,992,158), Japanese Patent Publication Laying-Open No. 55-164356 (U.S. Patent. No. 4,292,272), Japanese Patent Publication Laying-Open No. 54-101398 (U.S. Patent. No. 4,132,528) and the like. The thickness of the reagent layer comprising hydrophilic polymers as binders is about 2 µm to about 50 µm, preferably about 4 µm to about 30 µm on a dry basis, and the coverage is about 2 g/m² to about 50 g/m², preferably about 4 g/m² to about 30 g/m².

The reagent layer can further comprise an enzyme activator, a coenzyme, a surfactant, a pH buffer composition, an impalpable powder, an antioxidant, and various additives comprising organic or inorganic substances in addition to the reagent composition represented by formula 2 or 3 in order to improve coating properties and other various properties of diffusible compounds such as diffusibility, reactivity, and storage properties. Examples of buffers which can be contained in the reagent layer include pH buffer systems described in "Kagaku Binran Kiso (Handbook on Chemistry, Basic)," The Chemical Society of Japan (ed.), Maruzen Co., Ltd. (1996), p.1312-1320, "Data for Biochemical Research, Second Edition, R. M. C. Dawson et al. (2^{nd} ed.), Oxford at the Clarendon Press (1969), p. 476-508, "Biochemistry" 5, p. 467-477 (1966), and "Analytical Biochemistry" 104, p. 300-310 (1980). Specific examples of pH buffer systems include a buffer containing borate; a buffer containing citric acid or citrate; a buffer containing glycine, a buffer containing bicine; a buffer containing HEPES; and Good's buffers such as a buffer containing MES. A buffer containing phosphate cannot be used for a dry analytical element for detecting pyrophosphoric acid.

The dry analytical element for quantifying pyrophosphoric acid which can be used in the present invention can be prepared in accordance with a known method disclosed in the above-described various patent specifications. The dry analytical element for quantifying pyrophosphoric acid is cut into small fragments, such as, an about 5 mm to about 30 mm-square or a circle having substantially the same size, accommodated in the slide frame described in, for example, Japanese Patent Examined Publication No. 57-283331 (U.S. Patent. No. 4,169,751), Japanese Utility Model Publication Laying-Open No. 56-142454 (U.S. Patent. No. 4,387,990), Japanese Patent Publication Laying-Open No. 57-63452, Japanese Utility Model Publication Laying-Open No. 58-32350, and Japanese Patent Publication Laying-Open No. 58-501144 (International Publication WO 083/00391), and used as slides for chemical analysis. This is preferable from the viewpoints of production, packaging, transportation, storage, measuring operation, and the like. Depending on its intended use, the analytical element can be accommodated as a long tape in a cassette or magazine, as small pieces accommodated in a container having an opening, as small pieces applied onto or accommodated in an open card, or as small pieces cut to be used in that state.

The dry analytical element for quantifying pyrophosphoric acid which can be used in the present invention can quantitatively detect pyrophosphoric acid which is a test substance in a liquid sample, by operations similar to that described in the above-described patent specifications and the like. For example, about 2 µL to about 30 µL, preferably 4 µL to 15 µL of aqueous liquid sample solution is spotted on the reagent layer. The spotted analytical element is incubated at constant temperature of about 20°C to about 45°C, preferably about 30°C to about 40°C for 1 to 10 minutes. Coloring or discoloration in the analytical element is measured by the reflection from the light-transmissive support side, and the amount of pyrophosphoric acid in the specimen can be determined based on the principle of colorimetry using the previously prepared calibration curve. Quantitative analysis can be carried out with high accuracy by keeping the amount of liquid sample to be spotted, the incubation time, and the temperate at constant levels.

Quantitative analysis can be carried out with high accuracy in a very simple operation using chemical analyzers described in, for example, Japanese Patent Publication Laying-Open No. 60-125543, Japanese Patent Publication Laying-Open No. 60-220862, Japanese Patent Publication Laying-Open No. 61-294367, and Japanese Patent Publication Laying-Open No. 58-161867 (U.S. Patent. No. 4,424,191). Semiquantitative measurement may be carried out by visually judging the level of coloring depending on the purpose and accuracy needed.

Since the dry analytical element for quantifying pyrophosphoric acid which can be used in the present invention is stored and kept in a dry state before analysis, it is not necessary that a reagent is prepared for each use, and stability of the reagent is generally higher in a dry state. Thus, in terms of simplicity and swiftness, it is better than a so-called wet process, which requires the preparation of the reagent solution for each use. It is also excellent as an examination method because highly accurate examination can be swiftly carried out with a very small amount of liquid sample.

The dry analytical element for quantifying inorganic phosphorus which can be used in the second aspect of the present invention can be prepared by removing pyrophosphatase from the reagent layer in the aforementioned dry analytical element for quantifying pyrophosphoric acid. The dry analytical element described in Japanese Patent Publication Laying-Open No. 7-197 can also be used. The dry analytical element for quantifying inorganic phosphorus is similar to the aforementioned dry analytical element for quantifying pyrophosphoric acid in its layer construction, method of production, and method of application, with the exception that the reagent layer does not comprise pyrophosphatase.

The present invention is described in more detail with reference to the following examples. However, the technical scope of the present invention is not limited by these examples.

### Examples

### Example 1: Comparison of the expression levels of gene (HSA gene) between different cells

### (1) Preparation of mRNAs from different cells

### (1-1) HL-60

FBS (10% (w/w)) and 2-mercaptoethanol (0.1% (w/w)) were added to RPMI 1640 to prepare a medium, and HL-60 cells were cultured therein. When the cells became confluent, mRNA was extracted and purified by using RNeasy Mini Kit (QIAGEN Inc.).

### (1-2) Human liver mRNA

mRNA derived from human liver was purchased (hliver: Biochain).

### (2) RT reaction

The above mRNA was subjected to RT reaction under the following reaction conditions to synthesize cDNA.

### <Composition of reaction solution>

| | |
|---|---|
| mRNA | 200 ng |
| Oligo dT₁₂₋₁₈ₘₑᵣ (Invitrogen) | 1 µl |
| 10 mM dNTPs | 1 µl |
| DEPC-dH₂O | balance (to bring the total amount to 14 µl) |

This reaction solution was incubated at 65°C for 5 minutes. 5X 1st Strand Buffer (4 µl) and RNaseOut (1 µl, Invitrogen) were added, the mixture was incubated at 42°C for 2 minutes, SuperScript II (1 µl) was added, the mixture was incubated at 42°C for 50 minutes and then at 70°C for 15 minutes, RNaseH (1 µl) was added, and the mixture was incubated at 37°C for 20 minutes. Thus, HL-60-derived cDNA and hliver-derived cDNA were prepared.

### (3) PCR

The β-actin gene (as an internal standard) and the human serum albumin (HSA) gene (for comparison) were subjected to amplification by PCR under the following conditions using cDNA derived from each cell prepared in (2) above.

### <Primers for HSA detection>

Primer (upper): 5'-GAAAAGTGGGCAGCAAATGTT-3' (SEQ ID NO: 1)

Primer (lower): 5'-TCCTCGGCAAAGCAGGTCTC -3' (SEQ ID NO: 2)

### < Primers for β-actin detection>

Primer (upper): 5'-TGGGACGACATGGAGAAAATC-3' (SEQ ID NO: 3)

Primer (lower): 5'-AGGAAGGAAGGCTGGAAGAGT-3' (SEQ ID NO: 4)

HSA and β-actin genes were detected by carrying out amplification by PCR using the reaction solution having the composition shown in Table 1 below and using HL-60-derived cDNA and hliver-derived cDNA.

**Table 1:**

| Composition of reaction solution | | |
|---|---|---|
| | HSA | β-Actin (control) |
| 10× PCR buffer | 5 µl | 5 µl |
| 2.5 mM dNTP | 5 µl | 5 µl |
| 5 µM primer (upper) | 2.5 µl | 2.5 µl |
| 5 µM primer (lower) | 2.5 µl | 2.5 µl |
| HS Taq (TaKaRa) | 0.5 µl | 0.5 µl |
| Each nucleic acid solution obtained in (2) | 1 µl | 1 µl |
| Purified water | 28.5 µl | 33.5 µl |
| Total amount | 50 µl | 50 µl |

Amplification by PCR was carried out by repeating 35 cycles of denaturing at 94°C for 20 seconds, annealing at 60°C for 30 seconds, and polymerase elongation at 72°C for 1 minute.

### (4) Preparation of a dry analytical element for pyrophosphoric acid quantification

A colorless transparent polyethylene terephthalate (PET) smooth film sheet (support) comprising a gelatin undercoating layer (thickness of 180 µm) was coated with an aqueous solution having composition (a) shown in Table 2 to the following coverage. The coating was then dried to provide a reagent layer.

**Table 2:**

| Composition (a) of aqueous solution for reagent layer | |
|---|---|
| Gelatin | 18.8 g/m² |
| p-Nonylphenoxy polyxydol (containing 10 glycidol units on average) (C₉H₁₉-Ph-O-(CH₂CH(OH)-CH₂-O)₁₀H) | 1.5 g/m² |
| Xanthosine | 1.96 g/m² |
| Peroxidase | 15,000 IU/m² |
| Xanthine oxidase | 13,600 IU/m² |
| Purine nucleoside phosphorylase | 3,400 IU/m² |
| Leuco dye (2-(3,5-dimethoxy-4-hydroxyphonyl)-4- phenethyl-5-(4-dimethylaminophenyl)imidazole) | 0.28 g/m² |
| Water (pH was adjusted to 6.8 with a diluted NaOH solution) | 136 g/m² |

This reagent layer was coated with an aqueous solution for an adhesive layer having composition (b) shown in Table 3 below to the following coverage. The coating was then dried to provide an adhesive layer.

**Table 3:**

| Composition (b) of aqueous solution for adhesive layer | |
|---|---|
| Gelatin | 3.1 g/m² |
| p-Nonylphenoxy polyxydol (containing 10 glycidol unit on average) (C₉H₁₉-Ph-O-(CH₂CH(OH)-CH₂-O)₁₀H) | 0.25 g/m² |
| Water | 59 g/m² |

Subsequently, water was supplied to the adhesive layer over its whole surface at 30 g/m² to allow the gelatin layer to swell. A broad textile fabric made of genuine polyester was laminated thereon by applying slight pressure in a substantially even manner to provide a porous developing layer.

The developing layer was then substantially evenly coated with an aqueous solution having composition (c) shown in Table 4 below to the following coverage. The coating was then dried, cut into a size of 13 mm x 14 mm, and accommodated into a plastic mounting material, thereby preparing a dry analytical element for pyrophosphoric acid quantification.

**Table 4:**

| Composition (c) of aqueous solution for developing layer | |
|---|---|
| HEPES | 2.3 g/m² |
| Sucrose | 5.0g/m² |
| Hydroxypropyl methylcellulose (methoxy group 19% to 24%, hydroxypropoxy group 4% to 12%) | 0.04 g/m² |
| Pyrophosphatase | 14,000 IU/m² |
| Water (pH was adjusted to 7.2 with a diluted NaOH solution) | 98.6 g/m² |

### (5) Detection using an analytical element for pyrophosphoric acid quantification

The solutions after amplification by PCR in (2) above were spot deposited as they were on the dry analytical element for pyrophosphoric acid quantification prepared in (3) above in amounts of 20 µl each, and the dry analytical element for pyrophosphoric acid quantification was incubated at 37°C for 5 minutes. Thereafter, the optical density of reflection (OD_{R}) was assayed at the wavelength of 650 nm from the support side. The results are shown in Table 5. Fig. 1 shows changes in OD_{R} with time when the PCR solution of the target gene of each cell was spotted on a slide.

**Table 5:**

| Optical density of reflection (OD_{R}) of the PCR solution derived from the target gene of each cell 5 minutes later | | |
|---|---|---|
| | Optical density of reflection (OD_{R}) 5 minutes later | |
| Type of cell | β-actin | HSA |
| No template | 0.433 | 0.434 |
| Hliver | 0.601 | 0.619 |
| HL-60 | 0.621 | 0.445 |

The formula (calibration curve) representing the relationship between the optical densities of reflection and the known concentration levels of pyrophosphoric acid was previously prepared. With the use of this calibration curve, the optical densities of reflection (OD_{R}) of the PCR solutions derived from target genes of the aforementioned cells 5 minutes later were converted to the corresponding amounts of pyrophosphoric acid. The results are shown in Table 6.

**Table 6:**

| Correlation between the level and the amount of pyrophosphoric acid generated (mM) in PCR | | | | | |
|---|---|---|---|---|---|
| Type of cell | β-actin | -No | HSA | -No | H/β |
| No template | 0.044 | 0.000 | 0.043 | 0.000 | |
| hliver | 0.137 | 0.093 | 0.203 | 0.160 | 1.72 |
| HL-60 | 0.150 | 0.106 | 0.045 | 0.002 | 0.02 |
| Remark 1: (-No) indicates a value obtained by subtracting the PPi value of "No template" from the PPi value of each cell. | | | | | |
| Remark 2: (H/β) indicates a value obtained by dividing (-No) of HSA by (-No) of β-actin for normalization. | | | | | |

The values normalized using the β-actin gene (as an internal standard), i.e., H/β, were compared. This revealed that the HSA gene was expressed more in the hliver than in HL-60. This indicates that comparison of the pyrophosphoric acid amounts after RT-PCR enables the detection of differences in gene expression levels between different cells.

### Industrial Applicability

According to the method for analysis of the present invention, expression levels of target gene can be quantitatively assayed. Further, the method for analysis of the present invention enables simple and rapid detection of expression levels of gene using a small apparatus, without requiring any special techniques, complicated operations, and special apparatuses.

## Claims

1. A method for analyzing expression levels of a target nucleic acid in a biological sample which comprises steps of:
(1) conducting polymerase elongation by reacting RNA from a biological sample or cDNA synthesized therefrom, at least one primer complementary with a part of the target nucleic acid sequence contained in the RNA, at least one deoxynucleoside triphosphate, and at least one polymerase to synthesize said target nucleic acid sequence; and
(2) detecting or quantifying pyrophosphoric acid which is generated upon the polymerase elongation.

2. The method according to claim 1 wherein the expression level of the target nucleic acid is analyzed by using RNA from two or more samples or cDNA synthesized therefrom.

3. The method according to claim 1 wherein the expression level of the target nucleic acid is analyzed by using a gene that ubiquitously exists in every cell as an internal standard and normalizing the expression level of the target nucleic acid by using it.

4. The method according to claim 3 wherein β-actin or GAPDH gene is used as the internal standard.

5. The method according to claim 1 wherein pyrophosphoric acid is detected by using colorimetry.

6. The method according to claim 1 wherein pyrophosphoric acid is detected by using a dry analytical element.

7. The method according to claim 6 wherein the dry analytical element is the one for pyrophosphoric acid quantification, which comprises a reagent layer containing xanthosine or inosine, pyrophosphatase, purine nucleoside phosphorylase, xanthine oxidase, peroxidase, and a color developer.

8. The method according to claim 1 wherein the polymerase is selected from the group consisting of DNA polymerase I, Klenow fragment of DNA polymerase I, Bst DNA polymerase, and reverse transcriptase.

9. The method according to claim 1 wherein pyrophosphoric acid is detected by enzymatically converting pyrophosphoric acid to inorganic phosphorus and then using a dry analytical element for inorganic phosphorus quantification which comprises a reagent layer containing xanthosine or inosine, purine nucleoside phosphorylase, xanthine oxidase, peroxidase, and a color developer.

10. The method according to claim 9 wherein the enzyme that converts pyrophosphoric acid to inorganic phosphorus is pyrophosphatase.

11. The method according to claim 9 wherein the polymerase is selected from the group consisting of DNA polymerase I, Klenow fragment of DNA polymerase I, Bst DNA polymerase, and reverse transcriptase.
